# EUROPEAN PATENT SPECIFICATION

(11) **EP 4 463 116 B1**
(45) Date of publication and mention of the grant of the patent: **30.09.2026**
(21) Application number: 23740167.4
(22) Date of filing: 12.01.2023
(51) Int. Cl.: A61F 9/007

(54) **SHUNT FOR TREATING GLAUCOMA**
SHUNT ZUR BEHANDLUNG VON GLAUKOM
SHUNT POUR TRAITER LE GLAUCOME

(30) Priority: 14.01.2022 ZA 202200672
(43) Date of publication of application: 20.11.2024
(73) Proprietor: Liqid Medical Proprietary Limited, Cape Town 7925 (ZA)
(72) Inventor: MCCLUNAN, Daemon Bruce, 7925 Cape Town (ZA); FISCHER, Joshua David, 7611 Cloetesville (ZA)
(74) Representative: Algemeen Octrooi- en Merkenbureau B.V.
(86) International application number: PCT/IB2023/050284
(87) International publication number: WO 2023/135547

(56) References cited:
- EP-A1- 3 666 236
- WO-A1-2021/250609
- WO-A2-2014/043698
- US-A- 5 882 327
- US-B2- 11 246 753
- US-B2- 8 702 639
- US-B2- 8 702 639
- US-B2- 8 721 656
- US-B2- 9 707 128
- US-B2- 9 707 128

## Description

### TECHNICAL FIELD

This invention relates to a shunt for treating glaucoma in a patient. More specifically, the invention relates to a shunt for treating glaucoma in a patient by diverting aqueous fluid from a chamber of the eye to the subconjunctival space of the patient.

### BACKGROUND TO INVENTION

Glaucoma is an ocular disease characterised by the presence of raised intraocular pressure (IOP) causing irreversible damage to the optic nerve. The ocular globe of the eye has a tough outer layer comprised of the sclera and the cornea. The internal areas of the eye are separated into the anterior segment and the posterior segment. The anterior segment comprises the anterior and posterior chambers of the eye filled with aqueous fluid, and the posterior segment comprises the vitreous chamber filled with vitreous gel. The cornea merges into the sclera at a juncture referred to as the limbus. A portion of the sclera is covered by a thin tissue called Tenon's membrane (also called Tenon's capsule), which envelopes the bulb of the eye from the optic nerve to the ciliary region. A portion of the Tenon's membrane is covered by another thin tissue membrane known as the conjunctiva. Near its front, Tenon's membrane blends into the conjunctiva where it is attached to the ciliary region of the eye.

The ocular globe maintains an internal pressure known as the intraocular pressure which normally varies between 10mmHg and 21mmHg. The intraocular pressure needs to be controlled within a defined range in order for the eye to function normally. The intraocular pressure is regulated by maintaining a balance between volumes of aqueous fluid produced and drained from the anterior segment of the ocular globe. Aqueous fluid is produced at a rate which varies between 2 to 3 microlitres per minute by the ciliary body. Age is one factor which affects the aqueous production rate, with elderly patients having a significantly lower aqueous production rate than younger patients. Aqueous fluid is drained from the anterior chamber through the trabecular and uveoscleral pathways at variable rates. If an impairment occurs in the amount of aqueous fluid drained from the ocular globe, then the intraocular pressure becomes too high. The presence of raised intraocular pressure results in a large pressure differential across the lamina cribrosa (translaminar pressure). This causes damage to the optic nerve head known as glaucoma. Glaucoma causes irreversible visual field defects. These defects enlarge until a patient's field of view is severely restricted. In the end stage of the disease, total vision loss occurs. Glaucoma is a leading cause of blindness worldwide. If the intraocular pressure remains very high, the eye can become persistently painful and may need to be removed.

Current medical, laser and surgical treatment options for glaucoma are aimed at lowering intraocular pressure. Glaucoma which is difficult to control through first line therapies such as topical medications and laser therapy is known as refractory glaucoma. Refractory glaucoma is often managed by glaucoma drainage tube implantation to create an additional aqueous outflow pathway from the anterior chamber into the subconjunctival space. Aqueous fluid draining into the subconjunctival space creates a fluid blister between the sclera and conjunctiva known as a bleb. Over time, the bleb becomes encapsulated by a fibrovascular wall of Tenons tissue.

In the early weeks following implantation, the bleb wall is not well formed and resistance to fluid flowing into the subconjunctival space is minimal. This means that glaucoma drainage devices tend to over drain in the early stages. Due to over drainage in the early stages, the IOP may drop below 5mmHg. This causes a condition known as hypotony. Hypotony may cause complications such as maculopathy and choroidal effusion.

The resistance to flow into the bleb then gradually increases during bleb wall formation in the intermediate period between 4 and 12 weeks following implantation. It is therefore preferable for devices to provide higher resistance to fluid flow in the early period following implantation to prevent hypotony, and lower resistance to flow in the intermediate to later stages to increase aqueous drainage.

Generally, the size of the bleb relates to the capacity of the bleb to absorb aqueous fluid. In the later stages following device implantation, the size of the bleb may reduce if inflammation and scarring occur due to unhealthy conjunctiva. If the size of the bleb is sufficiently reduced, then filtration failure and the recurrence of glaucoma may occur. In the presence of localised areas of unhealthy conjunctiva, the ideal bleb position for preventing filtration failure may differ between patients. The ideal bleb position may be as close as 4mm to the limbus or as far posteriorly as 30mm from the limbus. If the conjunctiva is generally unhealthy, a sub-Tenon's footplate connected to the drainage tube may be required to maintain the surface area of the bleb and help prevent filtration failure.

The presence of a tube in the anterior chamber is known to be a risk for damage to corneal endothelial cells which may result in corneal decompensation, vision loss and eventually a painful condition known as bullous keratopathy. The diameter, length, stiffness, and position of the tube in the anterior chamber are all known to contribute to the risk of endothelial cell damage.

If the tube is not securely fixed to the ocular globe then device migration may occur. Device migration may result in the tube becoming dislodged from within the anterior chamber or damage to endothelial cells.

Traditional aqueous drainage devices such as the Baerveldt device (US Patent 6,050,970) consist of a continuous diameter silicone tube with an outer diameter of about 0.6mm and internal diameter of about 0.3mm attached to a large footplate. The footplate is sutured to the sclera in the subconjunctival space at a position 10mm behind the limbus. A scleral channel which extends from the scleral surface to the anterior segment is then created using a needle body. The silicone tube is inserted through the scleral channel to enter the ocular anterior chamber. The silicone tube is then either completely or partially occluded using sutures to limit fluid flow and regulate pressure. Newer aqueous drainage devices such as the MicroShunt device (US Patent 9,101,444) consists of a straight tube of about 8mm in length with integrated tabs spaced intermediate proximal and distal ends of the tube. The entire length of the device is straight and comprises a microcapillary lumen with an internal diameter of about 0.07mm which is dimensioned to provide resistance to fluid flow preventing IOP from falling below 5 mmHg. A scleral channel is created using a needle body to enter the anterior chamber. The distal end of the tube is passed through the scleral channel to enter the anterior chamber. The tabs are positioned in the scleral channel to create a fluid seal between the tube and surrounding scleral tissue. The proximal end is then left lying in the subconjunctival space to create a bleb roughly 6mm from the limbus.

International patent application WO 2014 043698 A2 discloses devices and methods related to implants for treating one or more physiological conditions of the eye. Some embodiments include an expandable ocular implant for implanting in an eye. The expandable implant can include an implant having an elongate tubular body and an expandable sheath securely adapted to a part of the implant. Some embodiments of the expandable sheath can have at least one expandable feature that can assist the expandable sheath in forming a compact and an expanded configuration.

United States patent application US 8 702 639 B2 discloses a method of treating glaucoma in an eye by managing fluid flow past an implanted shunt having an elastomeric plate and a non-valved elastomeric drainage tube. The plate is positioned over a sclera of the eye with an outflow end of the elastomeric drainage tube open to an outer face of the plate. An inflow end of the drainage tube tunnels through the sclera to the anterior chamber of the eye. The plate may have regions of greater propensity for cell adhesion alternating with regions of lesser cell adhesion. For example, regions of texturing around the plate or drainage tube may be provided to control the size of a bleb that forms over the implant. The effective surface area of the plate may be balanced against a number of fenestrations. The drainage tube has a reduced profile and may be shaped with a non-circular external cross-section to reduce its height. A scleral groove may be used to further reduce the height of the drainage tube on the sclera. A flow restrictor for the early post operative period will immediately lower the intraocular pressure (IOP) and simultaneously prevent hypotony.

European patent application EP 3 666 236 A1 discloses a glaucoma implant device with an elongate member with two tube-like sections of different diameter, the tube of larger diameter being provided with holes and at least one fixation flange. The smaller diameter tube can be cut at an appropriate length during the operation, and is introduced directly into the anterior chamber and fixed with surgical tape.

United States patent application US 9 707 128 B2 discloses a method and shunt device for treating glaucoma including positioning a first end portion of a tube body into the retrobulbar space of an eye. The method also includes positioning a second end portion of the tube body into the anterior chamber of the eye. The tube body provides fluid passage for aqueous humor such that the aqueous humor flows from the first end portion into the retrobulbar space. A surgical kit for reducing the intraocular pressure in the eye includes the shunt device, an introducer, and a stylet. The introducer has a distal end for positioning in the retrobulbar space and a proximal end for positioning exterior of the eye. The introducer has a radius of curvature adapted to circumvent the eye for insertion into the retrobulbar space.

Existing glaucoma drainage devices suffer from a number of shortcomings which may increase the risk of aqueous fluid leakage, hypotony, filtration failure, endothelial cell damage and device migration. These shortcomings include:
1. Drainage devices such as the Baerveldt device and the Microshunt device create a bleb at a pre-determined distance from the limbus. This prevents the surgeon from being able to create a bleb at a position of their choosing where they feel it will have the lowest risk of scarring and filtration failure.
2. Drainage devices such as the Microshunt device include a capillary valve which traverses the entire length of the drainage device. This means the devices cannot be cut to the required length without significantly altering the resistance to fluid flow.
3. Drainage devices such as the Baerveldt and MicroShunt devices comprise straight tubing for traversing a scleral channel to enter the anterior chamber. This means that the direction of the tubing is aimed towards the corneal endothelium which is a risk for endothelial cell damage. It is desirable for tubing to travel in a direction parallel to the iris plane when entering the anterior or posterior chamber of the eye.
4. Drainage devices such as the Baerveldt device do not include a built-in flow resistance mechanism such as a leaf or capillary valve. This requires the surgeon to tie off the tubing using sutures for a period of time until the bleb has formed. This makes surgery more complex and delays the pressure lowering effect of such devices.
5. Drainage devices such as the MicroShunt device include locating tabs having rounded symmetrical outer profiles which provide minimal resistance to migration when implanted in a scleral channel. This means that relatively small longitudinal forces applied to the tubing can result in unwanted migration of the device along the scleral channel.
6. Drainage devices such as the Baerveldt and MicroShunt are provided either with, or without, a footplate respectively which means the surgeon cannot choose whether or not they feel a footplate is indicated for the specific patient.
7. Drainage devices such as the Baerveldt and MicroShunt provide a fixed resistance to fluid flow which cannot be tailored to the individual patient's aqueous production rate or allowed to vary depending on time after surgery.

It is an object of the present invention to provide a shunt for treating glaucoma which addresses the abovementioned shortcomings.

In this specification, the term "distal" means in the direction of the eye of a patient or away from a user of the shunt, while the term "proximal" means in the direction away from the eye of the patient or towards the user of the shunt.

### SUMMARY OF INVENTION

According to a first aspect of the invention the invention there is provided a shunt for treating glaucoma by lowering intraocular pressure in an eye of a patient according to claim 1.

The chamber of the eye in which the shunt is implanted may be the anterior chamber or the posterior chamber or the vitreous chamber of the eye.

The shunt may be configured to resist aqueous fluid flow at flow rates of between 1.5 to 3.0 microlitres per minute through the shunt using the Haigen Pouseille equation and a viscosity factor of 7.042 cP. More specifically, the shunt may be configured to resist aqueous fluid flow at flow rates of about 2 microlitres per minute through the shunt.

The distal lumen may have a diameter of between 0.12mm and 0.3mm. More specifically, the distal lumen may have a diameter of about 0.2mm.

The proximal capillary lumen may have a diameter of between 0.035mm and 0.06mm. More specifically, the diameter of the proximal capillary lumen may be about 0.05mm.

The distal portion may have a length of between 4mm and 30mm after cutting by the surgeon. The proximal portion may have a length of between 1mm and 8mm. More specifically, the proximal portion may have a length of around 5mm.

The distal portion may be configured to provide negligible fluid flow resistance less than 1mmHg while the proximal capillary portion may be configured to provide significant fluid flow resistance of between 4 mmHg and 12 mmHg.

The internal diameter of the distal lumen may be relatively larger than an internal diameter of the proximal lumen in a ratio of between 2 and 8 times larger.

The length of the distal portion may be relatively longer than the length of the proximal portion in a ratio of between 2 and 30 times longer.

In a second embodiment of the elongate duct, the proximal portion may comprise an inner wall and an outer wall, wherein the inner wall comprises a dissolvable substance which dissolves over a period of between 4 and 12 weeks, in order to facilitate adjustment of internal resistance to fluid flow through the proximal portion of the elongate duct.

The proximal portion of the elongate duct may be releasably connected to the distal portion of the elongate duct. Alternatively, the proximal portion of the elongate duct may be fixedly connected to the distal portion of elongate duct.

The shunt may include a scleral footplate which is operatively connected to the proximal end of the elongate duct for fixing the proximal end of the elongate duct to the sclera at the location of formation of the bleb and for enlarging the surface area over which aqueous fluid drains.

The proximal end of the elongate duct may be releasably connected to the footplate. Alternatively, the proximal end of the elongate duct may be fixedly connected to the footplate.

The proximal portion of the elongate duct may have a rigid construction. The proximal portion may have an oval shape when viewed in cross section. The proximal portion may have a curvature that conforms to an anatomical curvature of the ocular globe.

The fixation body may be frictionally located on the distal portion of the elongate duct in an arrangement wherein a coefficient of friction acting between the fixation body and the elongate duct is sufficient to adequately resist movement of the fixation body relative to the elongate duct when the elongate duct is implanted in the scleral channel of the patient yet permits sliding displacement of the fixation body relative to the elongate duct when a moderate force is applied to the fixation body by a surgeon.

The internal passage of the fixation body and the distal portion of the elongate duct may be cylindrical.

The fixation body may have a pair of laterally-extending flanges which project outwardly from opposite sides thereof.

The fixation body may have a convexly rounded lower surface and a substantially flat upper surface.

The fixation body may be of a rigid construction.

The fixation body may have an upper body portion which engages an upper side of the distal portion and a lower body portion which engages a lower side of the distal portion of the elongate duct, the upper body portion being relatively wider than the lower body when viewed in side view. The asymmetrical shape of the fixation body resists displacement of the shunt within a scleral channel created within scleral tissue. The asymmetrical shape furthermore causes bending in the distal portion at regions thereof adjacent opposite proximal and distal sides of the fixation body so as to direct the distal end away from the corneal endothelium and towards the iris plane.

In a particular embodiment of the fixation body, the fixation body may define a curved internal passage in which the elongate duct is received. The curvature of the internal passage causes bending of the elongate duct received therein, thereby resisting displacement of the shunt within a scleral channel created within scleral tissue thereby to resist displacement of the shunt within a scleral channel created within scleral tissue and direct the distal end away from the corneal endothelium and towards the iris plane of the patient.

In a third embodiment of the elongate duct, the distal portion may comprise a wall thickness or diameter which varies along the length of the tube in order to facilitate adjustment of the internal resistance to fluid flow through the distal portion. More specifically, in a first example of the third embodiment, the outer diameter of the distal portion may taper along at least along a portion of the length of the distal portion, with the wall thickness remaining constant, providing the lumen of the distal portion with a tapered configuration. In a second example of the third embodiment, the wall thickness of the distal portion may taper along at least a portion of the length of the distal portion, with the outer diameter remaining constant, providing the lumen of the distal portion with a tapered configuration. In use, the internal resistance to fluid flow through the distal portion can be adjusted by moving the position of the fixation body along the distal portion in the region of the tapered lumen in order to provide for variations in patient aqueous fluid production rates.

Also disclosed herein is a surgical method for treating glaucoma in a patient by lowering intraocular pressure in an eye of a patient, which method is not part of the current invention. The surgical method comprises:
opening the conjunctival/tenon's complex to create a pocket between the conjunctival/tenon's complex and the sclera;
providing the shunt as defined and described hereinabove in accordance with the first aspect of the invention;
sliding the fixation body along the distal portion of the elongate duct until a desired fixation body position is achieved corresponding to a desired length of the distal portion of the elongate duct and a desired position of the fixation body along the elongate duct for fixing the distal portion at a desired position relative to the limbus;
cutting the distal portion of the elongate duct so as to adjust a length of the elongate duct;
using a surgical instrument, creating a passageway through scleral tissue so as to form a scleral channel from an external position at the pocket in the conjunctival/tenon's complex to the chamber of the eye from which aqueous fluid is to be diverted;
inserting the distal portion of the elongate duct of the shunt into the scleral channel until the distal end of the elongate duct lies within the chamber of the eye; and
closing the conjunctival/tenon's complex leaving the proximal end of the shunt lying within the pocket.

### BRIEF DESCRIPTION OF THE DRAWINGS

Further features of the invention are described in more detail hereinafter, by way of a non-limiting example of the invention, with reference to and as illustrated in the accompanying diagrammatic drawings. In the drawings:

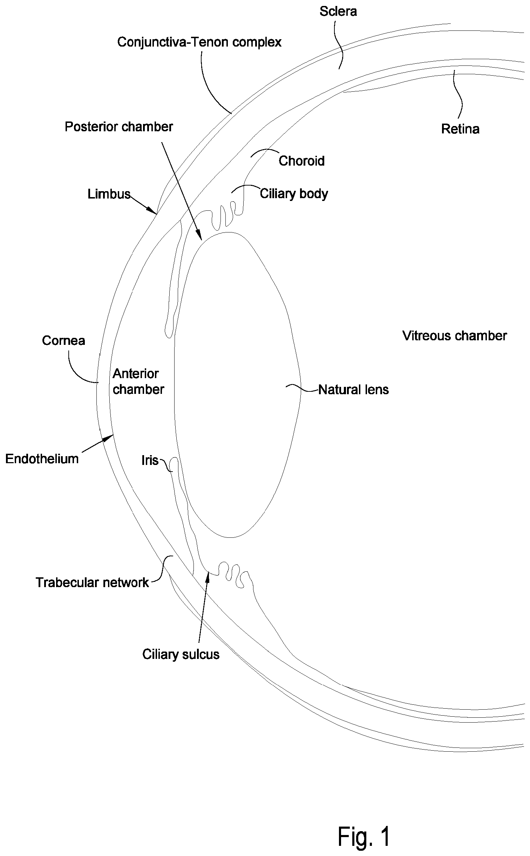

Figure 1 shows a prior art Illustration providing anatomic details of a human eye;
Figure 2 shows a side view of a shunt for treating glaucoma, in accordance with the invention, prior to cutting the shunt to a desired length;
Figure 3 shows top plan view of the shunt of Figure 2;
Figure 4 shows a distal end view of the shunt of Figure 2;
Figure 5 shows a proximal end view of the shunt of Figure 2;
Figure 6 shows a perspective view as seen from above of the fixation body of the shunt of Figure 2;
Figure 7 shows an end view of the fixation body;
Figure 8 shows an opposite end view of the fixation body;
Figure 9 shows a side view of the fixation body;
Figure 10 shows a sectional side view of the fixation body;
Figure 11 shows a side view of the shunt of Figure 2, illustrating the manner in which the fixation body is displaced along the distal portion of the elongate duct using forceps;
Figure 12 shows a side view of the shunt of Figure 2, illustrating the manner in which the distal portion of the elongate duct is cut by a surgeon;
Figure 13 shows a side view of the shunt of Figure 2 after it has been cut to a desired length;
Figure 14 shows a sectional side view of the shunt of Figure 13;
Figure 15 shows enlarged detail A of Figure 14;
Figure 16 illustrates the manner in which a channel is created in the sclera using a surgical instrument for insertion of the shunt of Figure 2 into the anterior chamber of the eye of the patient;
Figure 17 illustrates the shunt of Figure 2 wherein the distal portion is cut so as to provide a relatively short section of tubing and implanted in the anterior chamber of the patient's eye and creating a bleb near to the limbus;
Figure 18 shows enlarged detail B of the shunt of Figure 17;
Figure 19 shows the implanted shunt of Figure 2 wherein the distal portion is cut so as to provide a relatively long section of tubing and implanted in the anterior chamber of the patient's eye and creating a bleb away from the limbus;
Figure 20 shows the implanted shunt of Figure 17 with a shorter extension tube and a footplate connected thereto so as to adjust the size and position at which a bleb is formed;
Figure 21 illustrates the manner in which a channel is created in the sclera using a surgical instrument for insertion of the shunt of Figure 2 into the posterior chamber of the eye of the patient;
Figure 22 shows the shunt of Figure 2, implanted in the posterior chamber of the eye;
Figure 23 shows a perspective view as seen from above of another embodiment of a fixation body of the shunt in accordance with the invention;
Figure 24 shows a sectional side view of the fixation body of Figure 23;
Figure 25 shows a sectional side view of the shunt including the fixation body of Figure 23;
Figure 26 shows enlarged detail C of the shunt of Figure 25;
Figure 27 illustrates the manner in which a channel is created in the sclera using a surgical instrument for insertion of the shunt of Figure 2 into the vitreous chamber of the eye of the patient;
Figure 28 shows the shunt of Figure 2, implanted in the vitreous chamber of the eye; and
Figure 29 shows an enlarged fragmentary plan view of the proximal end of the elongate duct attached to a scleral footplate;
Figure 30 shows the manner in which the shunt of Figure 2 is attached to the scleral footplate of Figure 29;
Figure 31 shows the proximal end of the elongate duct of the shunt of Figure 2 attached to the scleral footplate of Figure 29, with the distal end implanted in the anterior chamber of the eye;
Figure 32 shows second embodiment of the elongate duct wherein the proximal portion has an inner wall and an outer wall and wherein the inner wall comprises a dissolvable substance;
Figure 33 shows a third embodiment of the elongate duct wherein the distal portion has a diameter which varies along the length of the distal portion; and
Figure 34 shows a third embodiment of the elongate duct wherein the distal portion has a wall thickness which varies along the length of the distal portion.

### DETAILED DESCRIPTION OF THE DRAWINGS

With reference to the drawings, a shunt for treating glaucoma by lowering intraocular pressure in an eye of a patient, is designated by the reference numeral 10.

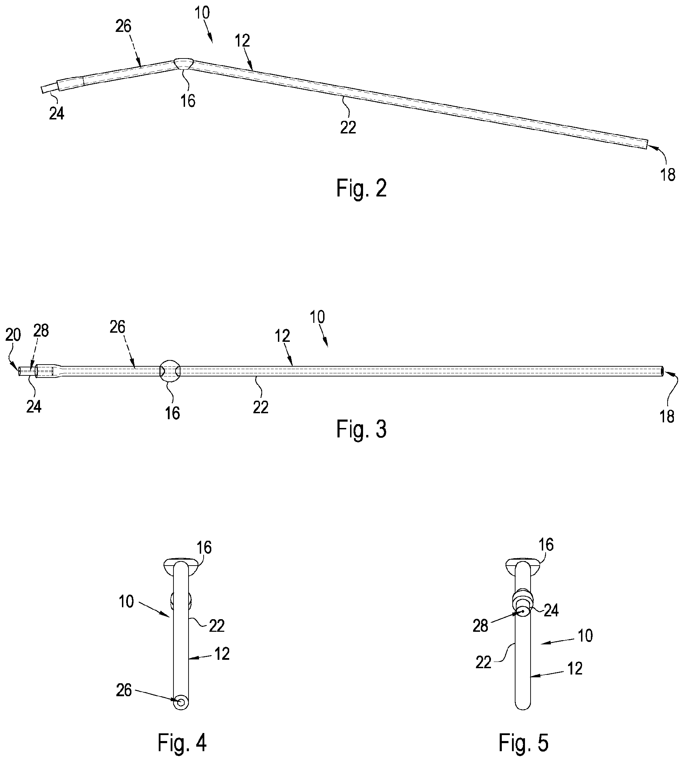

With reference to Figures 2-10, the shunt 10 comprises, broadly, an elongate silicone rubber duct 12 for defining a fluid passageway 14 for diverting aqueous humor from a chamber of the patient's eye and a fixation body 16 extending outwardly from the duct 12 for fixing the duct within tissue surrounding the eye. The relevant chamber of the eye may be the anterior chamber or the posterior chamber of the eye.

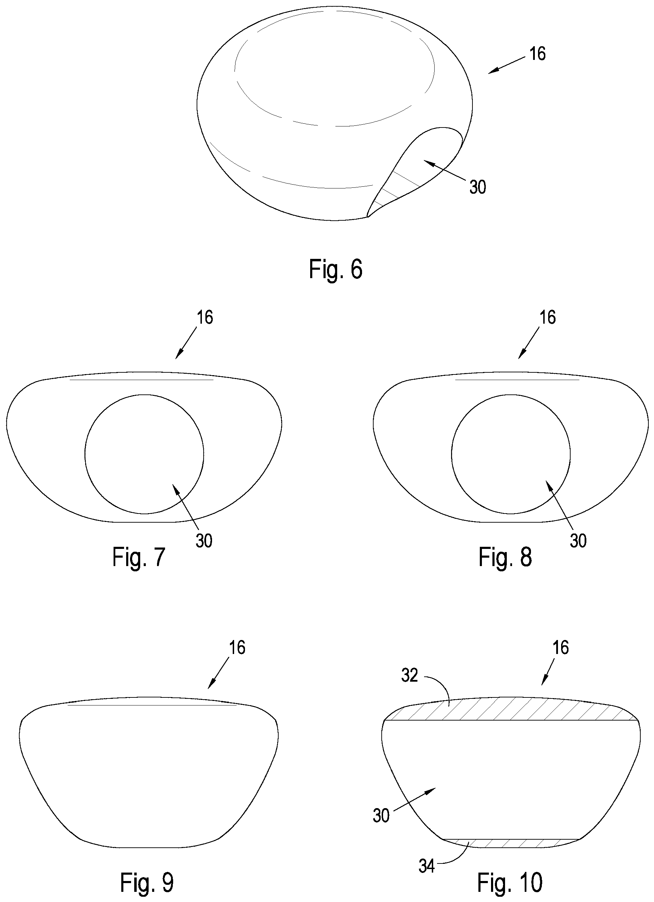

The elongate duct 12 has a distal end 18 and an opposite proximal end 20, the distal end 18 being implantable in the relevant chamber of the eye. The elongate duct has a distal portion 22 defining the distal end of the duct, which is locatable in the scleral channel of the patient, the distal portion being deformable so as to permit the distal portion to conform to anatomical structures of the eye of the patient. The distal portion is also severable, allowing a surgeon to cut the distal portion to a desired length corresponding to the anatomical dimensions of the patient's eye and required bleb position. As is illustrated in Figures 6-8, a bevel cut is made in the distal portion so as to define a relatively sharp point at a distal end 18.1 for facilitating insertion of the distal portion along a channel defined in scleral tissue.

The elongate duct 12 further has a proximal portion 24 having a rigid construction, which defines the proximal end of the elongate duct.

The distal portion 22 has a distal lumen 26 defining a distal part of the fluid passageway and the proximal portion 24 has a proximal capillary lumen 28 which is in fluid flow communication with the distal lumen, the proximal lumen having an internal diameter which is relatively smaller than an internal diameter of the distal lumen so as to reduce a flow rate of aqueous humor along the proximal capillary lumen.

The shunt is configured to resist aqueous fluid flow at flow rates of between 1.5 to 3.0 microlitres per minute through the shunt using the Haigen Pouseille equation and a viscosity factor of 7.042 cP and more specifically, about 2 microlitres per minute.

The distal lumen has a diameter of between 0.12mm and 0.3mm. More specifically, the distal lumen has a diameter of about 0.2mm.

The proximal capillary lumen has a diameter of between 0.035mm and 0.06mm. More specifically, the diameter of the proximal capillary lumen is about 0.05mm.

The distal portion has a length of between 4mm and 30mm after cutting by the surgeon.

The proximal portion has a length of between 1mm and 8mm. More specifically, the proximal portion has a length of around 5mm. The distal portion is configured to provide negligible fluid flow resistance less than 1mmHg while the proximal capillary portion is configured to provide significant fluid flow resistance of between 4 mmHg and 12 mmHg.

The internal diameter of the distal lumen is relatively larger than an internal diameter of the proximal lumen in a ratio of between 2 and 8 times larger.

The length of the distal portion is relatively longer than the length of the proximal portion in a ratio of between 2 and 30 times longer.

In a particular embodiment of the invention, the proximal portion of the elongate duct is releasably connected to the distal portion of the elongate duct. In another embodiment of the invention, the proximal portion of the elongate duct is fixedly connected to the distal portion of elongate duct.

The proximal portion of the elongate duct has an oval shape when viewed in cross section. The proximal portion has a curvature that conforms to that of an anatomical curvature of the ocular globe.

The fixation body 14 of the shunt forms an effective seal with surrounding scleral tissue. The fixation body is slidably located on the distal portion of the elongate duct 12 for fixing the distal portion 22 of the duct within a channel created in scleral tissue at a desired position determined by a required length of the shunt as will be explained in more detail hereinbelow.

The fixation body 14 is frictionally located on the distal portion 22 of the elongate duct in an arrangement wherein a coefficient of friction acting between the fixation body and the elongate duct is sufficient to adequately resist movement of the fixation body relative to the elongate duct following implantation yet permit sliding displacement of the fixation body relative to the elongate duct when a force is applied to the fixation body by a surgeon during implantation.

The fixation body defines an internal passage 30 within which the distal portion of the elongate duct is received. The internal passage 30 of the fixation body and the distal portion of the elongate duct are cylindrical. The fixation body has an upper body portion 32 which engages an upper side of the distal portion and a lower body portion 34 which engages a lower side of the distal portion, the upper body portion being relatively wider than the lower body when viewed in side view. The asymmetrical shape of the fixation body resists displacement of the shunt within a scleral channel created within scleral tissue and causes bending in the distal portion away from the endothelium.

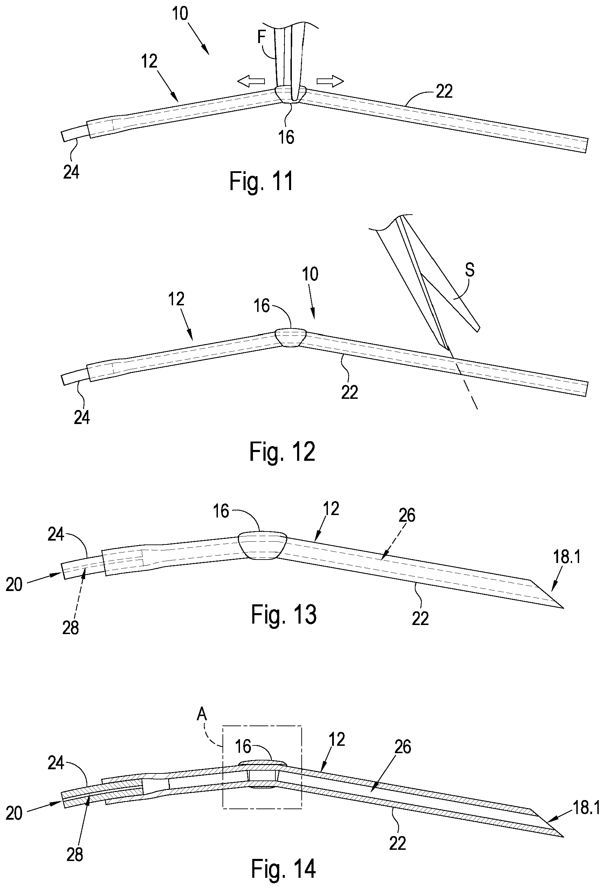

With reference to Figures 11-20, the manner in which the shunt 10 is implanted in the anterior chamber of an eye of a patient, is illustrated. The anatomical dimensions and ideal bleb location of the eye of the patient are initially measured by the surgeon performing a visual observation of the patient's eye in order to determine an optimal length for the shunt.

With specific reference to Figure 11, the fixation body 16 is then slid along the distal portion of the elongate duct by a surgeon gripping the fixation body 16 using forceps and applying a force to the fixation body in a direction for displacing the fixation body along the distal portion until a desired position along the distal portion is achieved. An important consideration in this regard is the position at which the surgeon would like to form a bleb into which aqueous humor from the anterior chamber is allowed to drain after implantation of the shunt.

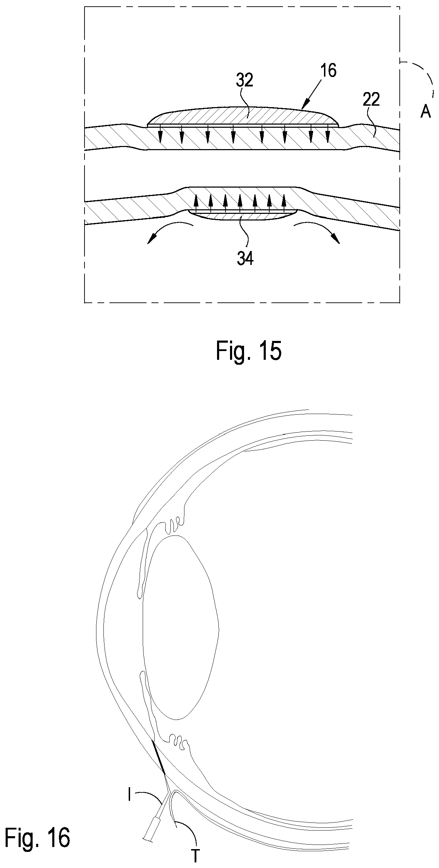

As is more clearly illustrated in Figure 15, an internal diameter of the internal passage 30 of the fixation body is slightly less than an external diameter of the distal portion of the elongate duct, causing the fixation body to be located on the distal portion 22 of the duct 12 in an interference fit permitting sliding displacement of the fixation body when sufficient force is applied to the fixation body in order to overcome frictional forces holding the fixation body on the distal position.

With reference to Figures 12-14, a distal portion 22 of the elongate duct 12 of the shunt is then cut by a surgeon using surgical scissors S, so as to adjust the length of the shunt to a desired length. More specifically, an angled bevel cut is made in the distal portion so as to provide a distal end 18.1 having a sharp point for facilitating insertion of the distal portion along the scleral channel and into the anterior or posterior chamber.

With reference to Figure 16, a portion T of the conjunctiva-tenon's complex is released from the limbus and retracted. A passageway is then created through scleral tissue by a surgeon using a surgical blade I so as to form a scleral channel from an external position having an entry point approximately 3mm from the limbus to an exit point into the anterior chamber at the trabecular network. The distal portion of the elongate duct of the shunt is then inserted into the scleral channel until the fixation body lies within the scleral channel and the distal end of the elongate duct lies within the anterior chamber.

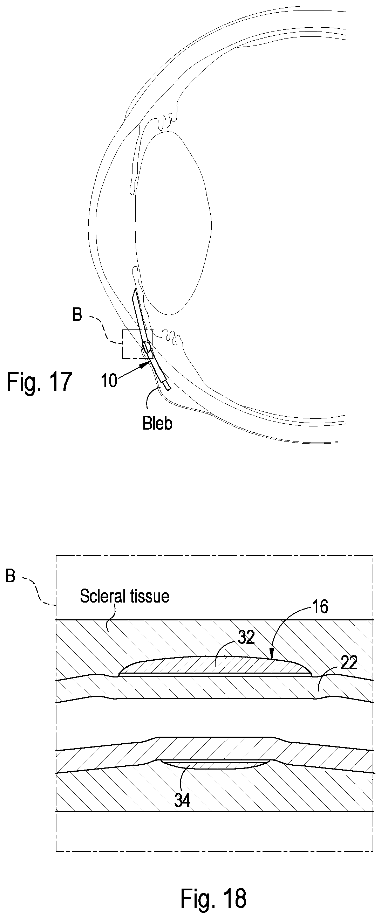

In Figure 17, the shunt 10 is shown implanted in the anterior chamber of the patient's eye, providing a passageway along which aqueous humor is drained from the anterior chamber into a bleb formed between scleral tissue and the conjunctiva-tenon's complex near to the limbus. In Figure 18, the manner in which the fixation body grips scleral tissue within the scleral channel, is illustrated.

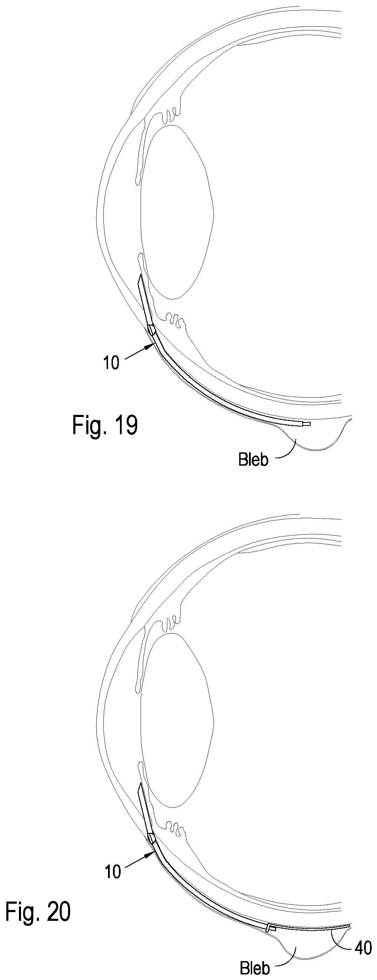

With reference to Figure 19, a shunt 10 wherein the fixation body has been adjusted to be closer to the distal end and the shunt has been cut short to effectively adjust the position at which a bleb is formed, is shown

With reference to Figure 20, the shunt 10 is shown with a Baerveldt device 40 attached thereto and located at a base of a bleb formed between scleral tissue and the conjunctiva-tenon's complex.

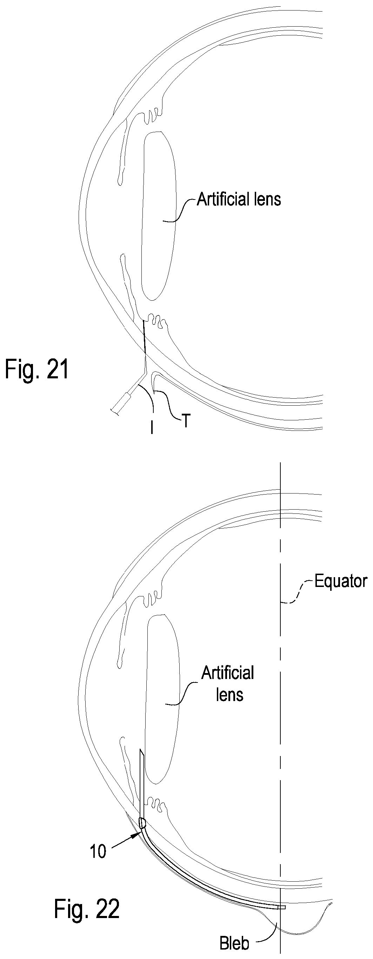

With reference to Figures 21 and 22, the manner in which the shunt 10 is implanted in the posterior chamber of an eye and used, is illustrated. The shunt 10 is implanted in the same manner as described above in relation to the implantation of the shunt into the anterior chamber of the eye, with the only difference being that the scleral channel is formed with an exit point into the posterior chamber at the ciliary sulcus.

The shunt 10 provides an extended length of deformable tube with a fixation body slidably located thereon so as to allow a surgeon to adjust the position of the fixation body along the elongate duct to thereby adjust the length of the shunt to the anatomical dimensions of the eye of a patient and provide for optimal positioning of a bleb into which aqueous humor can drain.

The proximal portion of the elongate duct provides the shunt with a capillary valve, while the distal portion of the elongate duct may be cut so as to adjust the length of the shunt without affecting the capillary valve or altering the resistance to fluid flow along the fluid passageway of the shunt significantly.

The interference fit of the fixation body on the distal portion of the shunt provides for resistance to migration of the fixation body along the elongate duct when the shunt is implanted, while allowing for movement along the distal portion in order to adjust the length of the shunt. The outwardly projecting formations furthermore create a fluid seal in the scleral channel.

The asymmetrical shape of the fixation body resists displacement of the shunt within a scleral channel created within scleral tissue and creates a bend away from the endothelium as described hereinabove.

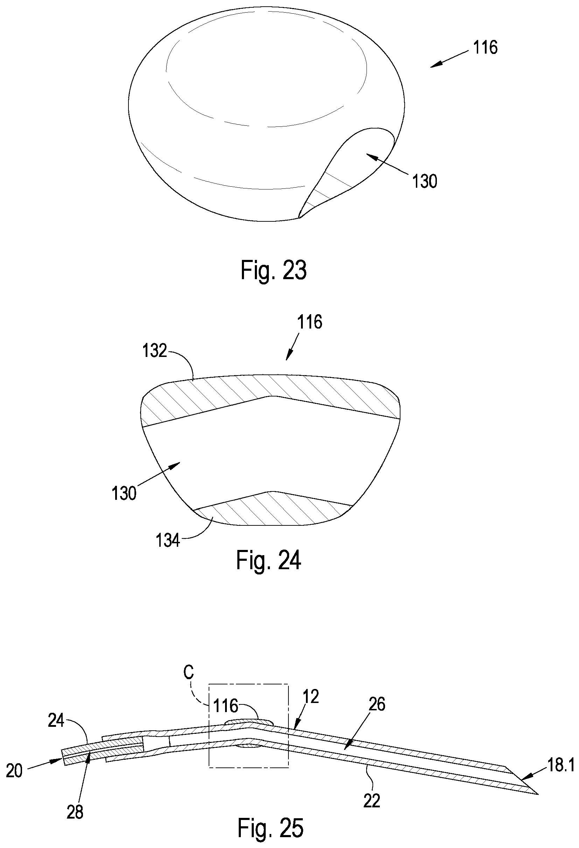

Figures 23 and 24 show another embodiment of a fixation body of the shunt in accordance with the invention, which is designated by the reference numeral 116. The fixation body 116 is similar to the fixation body and those features of the fixation body 116 which are the same as and/or the similar to those of the fixation body 16 are designated in Figures 23 and 24 by the same and/or similar reference numerals. The fixation body 116 defines a curved internal passage 130 in which the distal portion 22 of the elongate duct is received, the curvature of the internal passage 130 being configured so as to cause bending of the elongate duct when the elongate duct is received therein, thereby to resist displacement of the shunt within a scleral channel created within scleral tissue and direct the distal end away from the corneal endothelium and towards the iris plane of the patient.

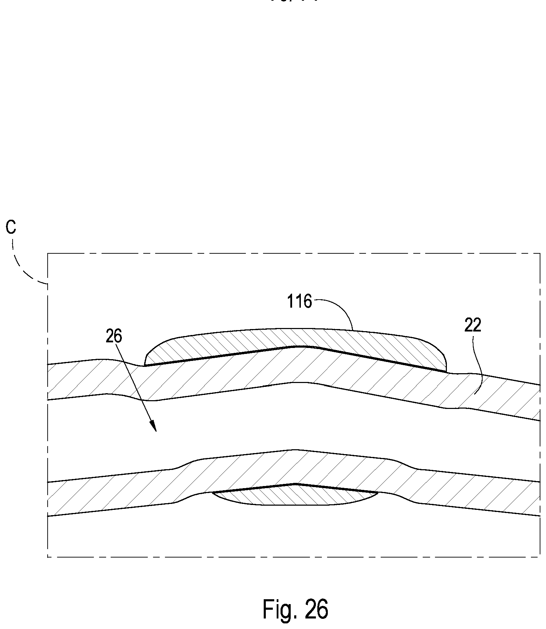

Figures 25 and 26 show a shunt in accordance with the invention, including the fixation body 116, illustrating the manner in which the elongate duct is bent by the fixation body.

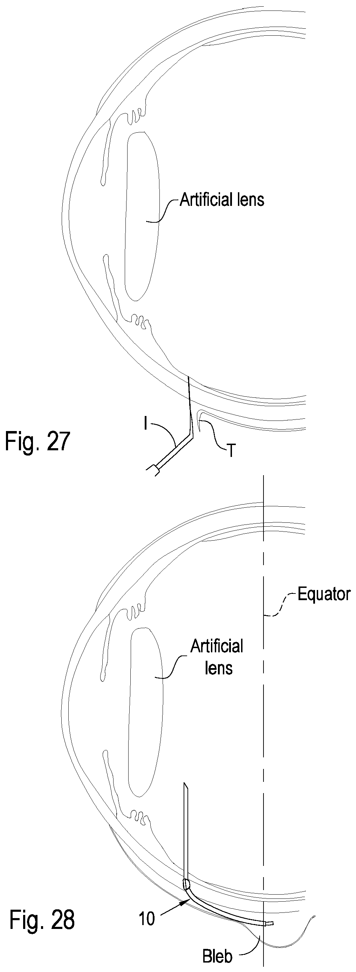

With reference to Figures 27 and 28, the manner in which the shunt 10 is implanted in the vitreous chamber of an eye and used, is illustrated. The shunt 10 is implanted in the same manner as described above in relation to the implantation of the shunt into the anterior chamber of the eye, with the only difference being that the scleral channel is formed in the pars plana region of the ciliary body with an exit point into the vitreous chamber.

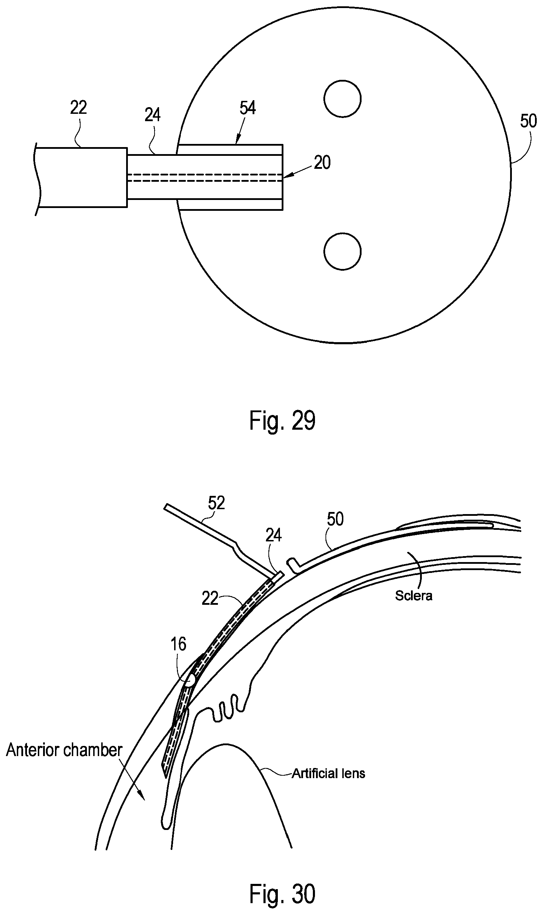

With reference to Figures 29 - 31, the shunt 10 includes a scleral footplate 50 which is operatively connected to the proximal end 20 of the elongate duct 12 for fixing the proximal end of the elongate duct to the sclera at the location of formation of a bleb. The proximal shunt portion 24 is gripped by a surgeon using forceps 52 and attached to a receiving recess 54 in the footplate. The proximal end of the elongate duct may be releasably connected to the footplate. Alternatively, the proximal end of the elongate duct may be fixedly connected to the footplate. The scleral footplate increases the surface area of the bleb in patients with generally unhealthy conjunctiva.

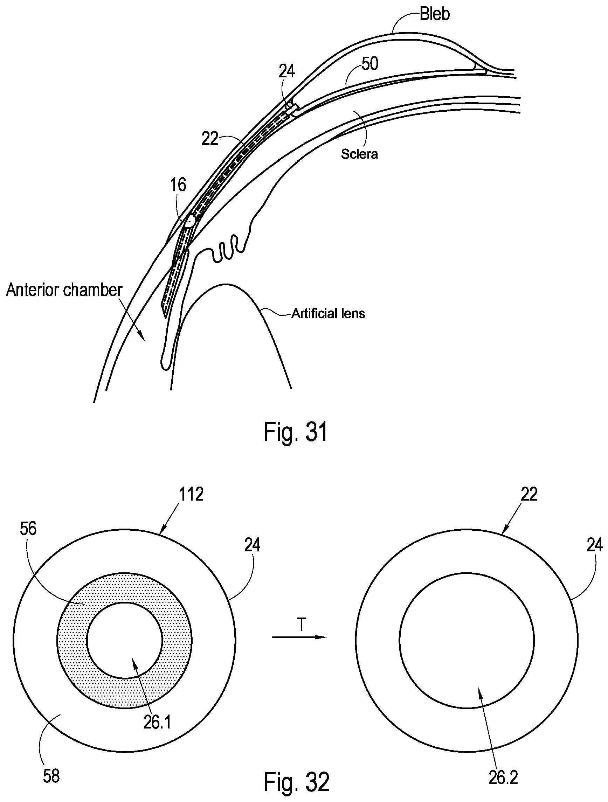

With reference to Figure 32, a second embodiment of the elongate duct is designated by the reference numeral 112. The duct 112 is similar to the elongate duct 12, with the only difference being that the proximal portion 24 initially comprises an inner wall 56 in addition to an outer wall 58, wherein the inner wall 56 comprises a dissolvable substance which dissolves over a period T of between 4 and 12 weeks, in order to facilitate adjustment of internal resistance to fluid flow through the proximal portion of the elongate duct. At the beginning of the period T, the inner wall provides a relatively high degree of resistance which gradually decreases as the inner wall dissolves until the inner wall is dissolved at the end of the period T as shown in Figure 32. The inner wall 56 defines a relatively narrow lumen 26.1 initially and after the inner wall has dissolved the outer wall defines a larger diameter lumen 26.2.

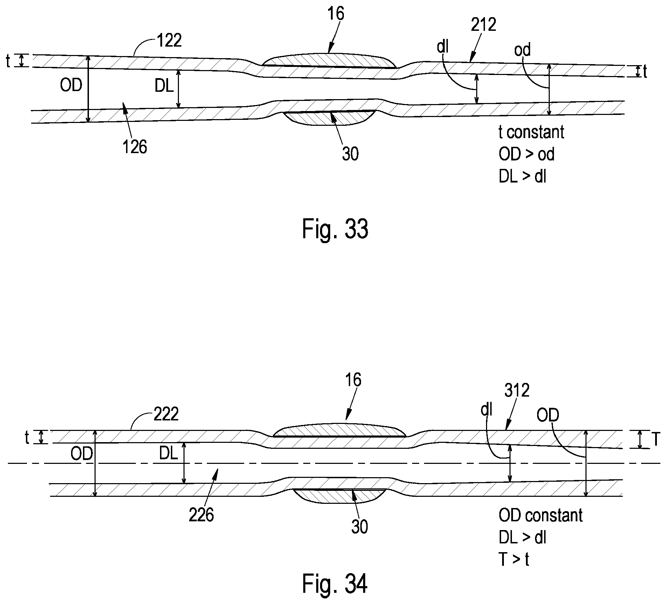

With reference to Figure 33, a first example of a third embodiment of the elongate duct is designated by the reference numeral 212. The elongate duct has a distal portion 122 having a constant wall thickness and an outer diameter which varies (from a relatively larger outer diameter OD to a relatively smaller outer diameter od) along the length of the distal portion. The outer diameter of the distal portion 122 is greater than the internal diameter of the internal passage 30 of the fixation body 16 such that when the distal portion 122 is received in the internal passage 30, the wall of the distal portion deforms inwardly. The diameter of the distal portion tapers along at least along a portion of the length of the distal portion, such that the lumen 126 has a tapered configuration. The diameter of the lumen 126 tapers from a relatively larger diameter DL to a relatively smaller diameter dl, with the wall thickness t remaining constant. In use, the internal resistance to fluid flow through the distal portion can be adjusted by moving the position of the fixation body along the distal portion in order to provide for variations in patient aqueous fluid production rates. The elongate duct 212 is pinched by the fixation body 16 when it is received in the internal passage 30 of the fixation body, causing inward deformation of the duct in the region of the fixation body and thereby narrowing of the lumen.

With reference to Figure 34, a second example of the third embodiment of the elongate duct is designated by the reference numeral 312. The elongate duct has a distal portion 222 having a constant outer diameter and a wall thickness which varies along the length of the tube (from a relatively large wall thickness T to a relatively small wall thickness t). The outer diameter of the distal portion 122 is greater than the internal diameter of the internal passage 30 of the fixation body such that when the distal portion 122 is received in the internal passage 30, the wall of the distal portion deforms inwardly. The wall thickness of the distal portion tapers in thickness along at least along a portion of the length of the distal portion. The diameter of the lumen 226 tapers from a relatively larger diameter DL to a relatively smaller diameter dl. In use, the internal resistance to fluid flow through the distal portion can be adjusted by moving the position of the fixation body along the distal portion in order to provide for variations in patient aqueous fluid production rates. The elongate duct 212 is pinched by the fixation body 16 when it is received in the internal passage 30 of the fixation body, causing inward deformation of the duct in the region of the fixation body and thereby narrowing of the lumen.

In summary, a surgical method for treating glaucoma in a patient by lowering intraocular pressure in an eye of a patient, which method is not part of the current invention, comprises:
providing the shunt 10 as defined and described hereinabove;
measuring the anatomical dimensions of the eye of the patient and a distance to a desired location for the formation of a bleb into which aqueous humor can drain, in order to determine an optimal length for the shunt;
sliding the fixation body 16, 116 along the distal portion 22 of the elongate duct 12 until a desired fixation body position is achieved corresponding to a desired length of the distal portion of the elongate duct and a desired position of the fixation body along the elongate duct for fixing the distal portion within the scleral channel;
cutting the distal portion of the elongate duct so as to adjust a length of the elongate duct;
opening the conjunctival/tenon's complex to create a pocket between the conjunctival/tenon's complex and the sclera;
using a surgical blade I, creating a passageway through scleral tissue so as to form a scleral channel from an external position at the pocket in the conjunctival/tenon's complex to the anterior/posterior chamber of the eye from which aqueous fluid is to be diverted;
inserting the distal portion of the elongate duct of the shunt into the scleral channel until the fixation body 16,116 lies within the scleral channel and the distal end 18.1 of the elongate duct lies within the anterior/posterior chamber of the eye; and
closing the conjunctival/tenon's complex leaving the proximal end of the shunt lying within the pocket.

The shunt and method described hereinabove ameliorate the shortcomings of the prior art devices described above.

## Claims

1. A shunt (10) for treating glaucoma by lowering intraocular pressure in an eye of a patient, the shunt comprising:
an elongate duct (12, 112, 212) defining a fluid passageway (14) for diverting aqueous humor from a chamber of the eye, the elongate duct having a distal end (18) and an opposite proximal end (20), the distal end (18, 18.1) being implantable in the chamber of the eye; and
a fixation body (16, 116) extending outwardly from the elongate duct,
the shunt being **characterized in that**:
the elongate duct has a distal portion (22, 122) defining the distal end of the duct, which is locatable in the scleral channel of the patient, the distal portion being deformable so as to permit the distal portion to conform to anatomical structures of the eye of the patient and being of a severable material configured to permit a surgeon to cut the distal portion to a desired length corresponding to a desired location for the formation of a bleb into which aqueous humor can drain; and a proximal portion (24), defining the proximal end of the elongate duct;
the distal portion has a distal lumen (26, 126) defining a distal part of the fluid passageway and the proximal portion has a proximal capillary lumen (28) which is in fluid flow communication with the distal lumen, the proximal lumen having an internal diameter which is relatively smaller than an internal diameter of the distal lumen so as to reduce a flow rate of aqueous humor and regulate pressure along the proximal capillary lumen sufficient to prevent hypotony; and
the fixation body of the shunt is slidably located on the distal portion of the elongate duct for fixing the distal portion of the duct within the scleral channel at a desired position determined by a required length of the shunt, wherein the fixation body defines an internal passage (30, 130) within which the elongate duct is received, and wherein the fixation body is located on the elongate duct in an interference fit wherein an internal diameter of the fixation body is slightly less than an external diameter of the distal portion of the elongate duct.

2. The shunt as claimed in claim 1, wherein the fixation body is frictionally located on the distal portion of the elongate duct in an arrangement wherein a coefficient of friction acting between the fixation body and the elongate duct is sufficient to adequately resist movement of the fixation body relative to the elongate duct when the elongate duct is implanted in the scleral channel of the patient yet permits sliding displacement of the fixation body relative to the elongate duct when a moderate force is applied to the fixation body by a surgeon.

3. The shunt as claimed in claim 1 or 2, wherein the fixation body (14) has an upper body portion (32) configured for engagement with an upper side of the distal portion and a lower body portion (34) configured for engagement with a lower side of the distal portion, the upper body portion being relatively wider than the lower body when viewed in side view, so as to cause bending of the elongate duct at regions thereof adjacent opposite proximal and distal sides of the fixation body when the distal portion of the elongate duct is received in the internal passage of the fixation body, thereby to resist displacement of the shunt within a scleral channel created within scleral tissue and direct the distal end away from the corneal endothelium and towards the iris plane of the patient.

4. The shunt as claimed in any one of the preceding claims, wherein the fixation body defines a curved internal passage in which the elongate duct is received, the curvature of the internal passage being configured so as to cause bending of the elongate duct when the elongate duct is received therein, thereby to resist displacement of the shunt within a scleral channel created within scleral tissue and direct the distal end away from the corneal endothelium and towards the iris plane of the patient.

5. The shunt as claimed in any one of the preceding claims, wherein, in order to facilitate adjustment of internal resistance to fluid flow through the proximal portion (24) of the elongate duct (16, 116), the proximal portion comprises an inner wall (56) and an outer wall (58) wherein the inner wall comprises a dissolvable substance which dissolves over a period of between 4 and 12 weeks.

6. The shunt as claimed in any one of the preceding claims, wherein, in order to facilitate adjustment of internal resistance to fluid flow through the distal portion of the elongate duct, the distal portion has a wall thickness or diameter which varies along the length of the distal portion so that the internal flow resistance can be adjusted by sliding the fixation body along the distal portion.

7. The shunt as claimed in any one of the preceding claims, wherein the shunt includes a scleral foot plate (50) which is operatively connected to the proximal end of the elongate duct, for fixing the proximal end of the elongate duct to the sclera at the location of formation of the bleb and increasing the surface area for aqueous fluid drainage.

8. The shunt as claimed in any one of the preceding claims, wherein the proximal portion of the elongate duct is releasably connected to the distal portion of the elongate duct.

9. The shunt as claimed in any one of the preceding claims 1 to 7, wherein the proximal portion of the elongate duct is fixedly connected to the distal portion of elongate duct.

10. The shunt as claimed in in any one of the preceding claims, wherein the proximal portion of the elongate duct has a rigid construction.

11. The shunt as claimed in any one of the preceding claims, wherein the proximal portion has an oval shape when viewed in cross section.

12. The shunt as claimed in any one of the preceding claims, wherein the proximal portion has a curvature that conforms to that of an anatomical curvature of the ocular globe.

13. The shunt as claimed in any one of the preceding claims, wherein the fixation body is of a rigid construction.

## Patentansprüche

1. Shunt (10) zur Behandlung von Glaukom durch Verringern des intraokularen Drucks in einem Auge eines Patienten, wobei der Shunt Folgendes umfasst:
einen länglichen Kanal (12, 112, 212), der einen Fluiddurchgang (14) zum Ableiten von Kammerwasser aus einer Kammer des Auges definiert, wobei der längliche Kanal ein distales Ende (18) und ein gegenüberliegendes proximales Ende (20) aufweist, wobei das distale Ende (18, 18.1) in die Kammer des Auges implantierbar ist; und
einen Befestigungskörper (16, 116), der sich von dem länglichen Kanal nach außen erstreckt,
wobei der Shunt **dadurch gekennzeichnet ist, dass**:
der längliche Kanal einen distalen Abschnitt (22, 122), der das distale Ende des Kanals definiert, der in dem skleralen Kanal des Patienten lokalisierbar ist, wobei der distale Abschnitt verformbar ist, um zu ermöglichen, dass sich der distale Abschnitt an anatomische Strukturen des Auges des Patienten anpasst, und aus einem abtrennbaren Material ist, das dazu konfiguriert ist, einem Chirurgen zu ermöglichen, den distalen Abschnitt auf eine gewünschte Länge entsprechend einer gewünschten Stelle für die Bildung eines Bläschens, in das Kammerwasser abfließen kann, zu schneiden; und einen proximalen Abschnitt (24), der das proximale Ende des länglichen Kanals definiert, aufweist;
der distale Abschnitt ein distales Lumen (26, 126) aufweist, das einen distalen Teil des Fluiddurchgangs definiert, und der proximale Abschnitt ein proximales kapillares Lumen (28) aufweist, das in Fluidflusskommunikation mit dem distalen Lumen ist, wobei das proximale Lumen einen internen Durchmesser aufweist, der relativ kleiner als ein interner Durchmesser des distalen Lumens ist, um eine Flussrate von Kammerwasser zu reduzieren und Druck entlang des proximalen kapillaren Lumens zu regulieren, der ausreicht, um Hypotonie zu verhindern; und
sich der Befestigungskörper des Shunts verschiebbar an dem distalen Abschnitt des länglichen Kanals befindet, um den distalen Abschnitt des Kanals innerhalb des skleralen Kanals an einer gewünschten Position zu befestigen, die durch eine erforderliche Länge des Shunts bestimmt ist, wobei der Befestigungskörper einen internen Durchgang (30, 130) definiert, innerhalb dessen der längliche Kanal aufgenommen ist, und wobei sich der Befestigungskörper an dem länglichen Kanal in einer Presspassung befindet, wobei ein interner Durchmesser des Befestigungskörpers geringfügig weniger als ein Außendurchmesser des distalen Abschnittes des länglichen Kanals ist.

2. Shunt nach Anspruch 1, wobei sich der Befestigungskörper reibschlüssig an dem distalen Abschnitt des länglichen Kanals in einer Anordnung befindet, wobei ein Reibungskoeffizient, der zwischen dem Befestigungskörper und dem länglichen Kanal wirkt, ausreichend ist, um Bewegung des Befestigungskörpers relativ zu dem länglichen Kanal angemessen zu widerstehen, wenn der längliche Kanal in dem skleralen Kanal des Patienten implantiert ist, jedoch Verschiebeversetzung des Befestigungskörpers relativ zu dem länglichen Kanal zulässt, wenn eine moderate Kraft auf den Befestigungskörper durch einen Chirurgen aufgebracht wird.

3. Shunt nach Anspruch 1 oder 2, wobei der Befestigungskörper (14) einen oberen Körperabschnitt (32), der zum Eingriff mit einer Oberseite des distalen Abschnittes konfiguriert ist, und einen unteren Körperabschnitt (34), der zum Eingriff mit einer Unterseite des distalen Abschnittes konfiguriert ist, aufweist, wobei der obere Körperabschnitt relativ breiter als der untere Körper ist, wenn in Seitenansicht gesehen, um Biegen des länglichen Kanals an Regionen davon benachbart zu gegenüberliegenden proximalen und distalen Seiten des Befestigungskörpers zu bewirken, wenn der distale Abschnitt des länglichen Kanals in dem internen Durchgang des Befestigungskörpers aufgenommen ist, um dadurch Versetzung des Shunts innerhalb eines skleralen Kanals, der innerhalb von skleralem Gewebe erzeugt wird, zu widerstehen und das distale Ende weg von dem Hornhautendothel und zu der Irisebene des Patienten zu lenken.

4. Shunt nach einem der vorhergehenden Ansprüche, wobei der Befestigungskörper einen gekrümmten internen Durchgang definiert, in dem der längliche Kanal aufgenommen ist, wobei die Krümmung des internen Durchgangs konfiguriert ist, um Biegung des länglichen Kanals zu bewirken, wenn der längliche Kanal darin aufgenommen ist, um dadurch Versetzung des Shunts innerhalb eines skleralen Kanals, der innerhalb von skleralem Gewebe erzeugt wird, zu widerstehen und das distale Ende weg von dem Hornhautendothel und zu der Irisebene des Patienten zu lenken.

5. Shunt nach einem der vorhergehenden Ansprüche, wobei, um Einstellung von internem Widerstand gegenüber Fluidfluss durch den proximalen Abschnitt (24) des länglichen Kanals (16, 116) zu erleichtern, der proximale Abschnitt eine Innenwand (56) und eine Außenwand (58) umfasst, wobei die Innenwand eine auflösbare Substanz umfasst, die sich über einen Zeitraum von zwischen 4 und 12 Wochen auflöst.

6. Shunt nach einem der vorhergehenden Ansprüche, wobei, um Einstellung von internem Widerstand gegenüber Fluidfluss durch den distalen Abschnitt des länglichen Kanals zu erleichtern, der distale Abschnitt eine Wanddicke oder einen Wanddurchmesser aufweist, die/der entlang der Länge des distalen Abschnittes variiert, sodass der interne Flusswiderstand durch Verschieben des Befestigungskörpers entlang des distalen Abschnittes eingestellt werden kann.

7. Shunt nach einem der vorhergehenden Ansprüche, wobei der Shunt eine sklerale Fußplatte (50) beinhaltet, die mit dem proximalen Ende des länglichen Kanals wirkverbunden ist, um das proximale Ende des länglichen Kanals an der Sklera an der Stelle der Bildung des Bläschens zu befestigen und den Oberflächenbereich für Abfluss von wässrigem Fluid zu erhöhen.

8. Shunt nach einem der vorhergehenden Ansprüche, wobei der proximale Abschnitt des länglichen Kanals lösbar mit dem distalen Abschnitt des länglichen Kanals verbunden ist.

9. Shunt nach einem der vorhergehenden Ansprüche 1 bis 7, wobei der proximale Abschnitt des länglichen Kanals fest mit dem distalen Abschnitt des länglichen Kanals verbunden ist.

10. Shunt nach einem der vorhergehenden Ansprüche, wobei der proximale Abschnitt des länglichen Kanals eine starre Konstruktion aufweist.

11. Shunt nach einem der vorhergehenden Ansprüche, wobei der proximale Abschnitt eine ovale Form aufweist, wenn im Querschnitt gesehen.

12. Shunt nach einem der vorhergehenden Ansprüche, wobei der proximale Abschnitt eine Krümmung aufweist, die sich an diejenige einer anatomischen Krümmung der okularen Globus anpasst.

13. Shunt nach einem der vorhergehenden Ansprüche, wobei der Befestigungskörper von einer starren Konstruktion ist.

## Revendications

1. Shunt (10) pour traiter le glaucome en abaissant la pression intraoculaire dans l'œil d'un patient, le shunt comprenant :
un conduit allongé (12, 112, 212) définissant une voie de passage de fluide (14) destiné à dévier l'humeur aqueuse provenant d'une chambre de l'œil, le conduit allongé possédant une extrémité distale (18) et une extrémité proximale opposée (20), l'extrémité distale (18, 18.1) étant implantable dans la chambre de l'œil ; et
un corps de fixation (16, 116) s'étendant vers l'extérieur à partir du conduit allongé,
le shunt étant **caractérisé en ce que** :
le conduit allongé possède une partie distale (22, 122) définissant l'extrémité distale du conduit, qui peut être située dans le canal scléral du patient, la partie distale étant déformable de manière à permettre à la partie distale de se conformer aux structures anatomiques de l'œil du patient et étant d'un matériau dissociable conçu pour permettre à un chirurgien de couper la partie distale à une longueur souhaitée correspondant à un emplacement souhaité pour la formation d'une bulle dans laquelle l'humeur aqueuse peut s'écouler ; et une partie proximale (24), définissant l'extrémité proximale du conduit allongé ;
la partie distale possède une lumière distale (26, 126) définissant une partie distale de la voie de passage de fluide et la partie proximale possédant une lumière capillaire proximale (28) qui est en communication d'écoulement fluidique avec la lumière distale, la lumière proximale présentant un diamètre interne qui est relativement plus petit que le diamètre interne de la lumière distale de manière à réduire le débit d'humeur aqueuse et à réguler la pression le long de la lumière capillaire proximale suffisamment pour empêcher l'hypotonie ; et
le corps de fixation du shunt est situé de manière coulissante sur la partie distale du conduit allongé pour fixer la partie distale du conduit à l'intérieur du canal scléral en une position souhaitée déterminée par la longueur requise du shunt, ledit corps de fixation définissant un passage interne (30, 130) à l'intérieur duquel le conduit allongé est reçu, et ledit corps de fixation étant situé sur le conduit allongé dans un ajustement serré dans lequel le diamètre interne du corps de fixation est légèrement inférieur au diamètre externe de la partie distale du conduit allongé.

2. Shunt selon la revendication 1, ledit corps de fixation étant situé par frottement sur la partie distale du conduit allongé dans un agencement dans lequel le coefficient de frottement agissant entre le corps de fixation et le conduit allongé est suffisant pour résister de manière adéquate au mouvement du corps de fixation par rapport au conduit allongé lorsque le conduit allongé est implanté dans le canal scléral du patient tout en permettant un déplacement coulissant du corps de fixation par rapport au conduit allongé lorsqu'une force modérée est appliquée sur le corps de fixation par un chirurgien.

3. Shunt selon la revendication 1 ou 2, ledit corps de fixation (14) possédant une partie de corps supérieure (32) conçue pour se mettre en prise avec un côté supérieur de la partie distale et une partie de corps inférieure (34) conçue pour se mettre en prise avec un côté inférieur de la partie distale, ladite partie de corps supérieure étant relativement plus large que le corps inférieur lorsqu'elle est vue de côté, de manière à provoquer la flexion du conduit allongé au niveau de ses zones adjacentes aux côtés proximal et distal opposés du corps de fixation lorsque la partie distale du conduit allongé est reçue dans le passage interne du corps de fixation, ce qui permet de résister au déplacement du shunt à l'intérieur d'un canal scléral créé à l'intérieur du tissu scléral et de diriger l'extrémité distale loin de l'endothélium cornéen et en direction du plan de l'iris du patient.

4. Shunt selon l'une quelconque des revendications précédentes, ledit corps de fixation définissant un passage interne incurvé dans lequel le conduit allongé est reçu, la courbure du passage interne étant conçue de manière à provoquer une flexion du conduit allongé lorsque le conduit allongé est reçu en son sein, ce qui permet de résister au déplacement du shunt à l'intérieur d'un canal scléral créé à l'intérieur du tissu scléral et de diriger l'extrémité distale à l'écart de l'endothélium cornéen et en direction du plan de l'iris du patient.

5. Shunt selon l'une quelconque des revendications précédentes, afin de faciliter le réglage de la résistance interne à l'écoulement de fluide à travers la partie proximale (24) du conduit allongé (12, 112), ladite partie proximale comprenant une paroi interne (56) et une paroi externe (58), ladite paroi interne comprenant une substance soluble qui se dissout sur une période comprise entre 4 et 12 semaines.

6. Shunt selon l'une quelconque des revendications précédentes, afin de faciliter le réglage de la résistance interne à l'écoulement de fluide à travers la partie distale du conduit allongé, ladite partie distale présentant une épaisseur ou un diamètre de paroi qui varie le long de la longueur de la partie distale de sorte que la résistance à l'écoulement interne puisse être réglée en faisant glisser le corps de fixation le long de la partie distale.

7. Shunt selon l'une quelconque des revendications précédentes, ledit shunt comprenant une plaque sclérale de base (50) qui est raccordée de manière fonctionnelle à l'extrémité proximale du conduit allongé, de manière à fixer l'extrémité proximale du conduit allongé à la sclère à l'emplacement de formation de la bulle et à augmenter la surface pour le drainage de fluide aqueux.

8. Shunt selon l'une quelconque des revendications précédentes, ladite partie proximale du conduit allongé étant raccordée de manière libérable à la partie distale du conduit allongé.

9. Shunt selon l'une quelconque des revendications précédentes 1 à 7, ladite partie proximale du conduit allongé étant raccordée de manière fixe à la partie distale du conduit allongé.

10. Shunt selon l'une quelconque des revendications précédentes, ladite partie proximale du conduit allongé présentant une construction rigide.

11. Shunt selon l'une quelconque des revendications précédentes, ladite partie proximale présentant une forme ovale lorsqu'elle est vue en coupe transversale.

12. Shunt selon l'une quelconque des revendications précédentes, ladite partie proximale présentant une courbure qui se conforme à celle de la courbure anatomique du globe oculaire.

13. Shunt selon l'une quelconque des revendications précédentes, ledit corps de fixation étant d'une construction rigide.
